(19) Europäisches Patentamt
European Patent Office
Office européen des brevets

(11) Publication number : **0 323 993 B1**

(12) # EUROPEAN PATENT SPECIFICATION

(45) Date of publication of patent specification : **30.09.92 Bulletin 92/40**

(51) Int. Cl.$^5$ : **A61L 31/00**

(21) Application number : **88905858.2**

(22) Date of filing : **05.07.88**

(86) International application number : **PCT/FI88/00108**

(87) International publication number : **WO 89/00431 26.01.89 Gazette 89/03**

(54) **ABSORBABLE MATERIAL FOR FIXATION OF TISSUES.**

(30) Priority : **10.07.87 FI 873047**

(43) Date of publication of application : **19.07.89 Bulletin 89/29**

(45) Publication of the grant of the patent : **30.09.92 Bulletin 92/40**

(84) Designated Contracting States : **AT BE CH DE FR GB IT LI LU NL SE**

(56) References cited :
**EP-A- 0 204 931**
**WO-A-87/00059**
**Biomaterials 1987, vol. 8 Jan. p 42-45 (P Törmälä et al), "the effects of fibre reinforcement and gold plating on theflexural and tensile strenght of PGA/PLA copolymer materials in vitro"**
**Biomaterials 1987, vol. 8 Jan. p 46-48 (S.Vainionpää et al), "Strength and strength retention in vitro, of absorbable,self-reinforced polyglycolide (PGA) rods for fracture fixation"**

(73) Proprietor : **BIOCON OY**
**Runeberginkatu 3 A 1**
**SF-33710 Tampere (FI)**

(72) Inventor : **TÖRMÄLÄ, Pertti**
**Runeberginkatu 3 A 1**
**SF-33710 Tampere (FI)**
Inventor : **MIKKONEN, Taito**
**Saarenmaantie**
**SF-36200 Kangasala (FI)**
Inventor : **LAIHO, Juha**
**Tapulintie 16 as. 3**
**SF-36200 Kangasala (FI)**
Inventor : **TAMMINMÄKI, Markku**
**Kukkolankatu 23 B 12**
**SF-33400 Tampere (FI)**
Inventor : **ROKKANEN, Pentti**
**Marjaniemenranta 29**
**SF-00930 Helsinki (FI)**
Inventor : **VAINIONPÄÄ, Seppo**
**Orapihlajatie 21-27 B 12**
**SF-00320 Helsinki (FI)**

(74) Representative : **Smulders, Theodorus A.H.J., Ir. et al**
**Vereenigde Octrooibureaux Nieuwe Parklaan 97**
**NL-2587 BN 's-Gravenhage (NL)**

**EP 0 323 993 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to synthetic, polymer-based surgical materials, which are absorbable (resorbable) in tissue conditions without causing harmful tissue reactions.

The manufacturing of sutures and osteosynthesis devices about absorbable polymers is known about several patents. The manufacturing of absorbable sutures and surgical elements of polyglycolide (PGA)

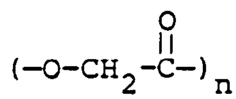

has been described in U.S. Pat. No. 3 297 033 and U.S. Pat. No. 3 739 773.

Sutures manufactured of polylactide (PLA)

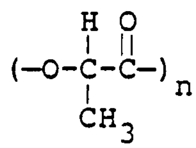

are described in U.S. Pat. No. 2 703 316.

Sutures manufactured of glycolide/lactide copolymers (PGA/PLA)

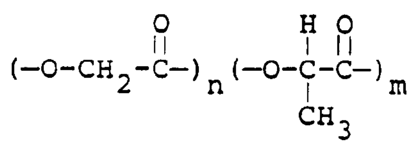

(where n and m are integers > 1) are described in U.S. Pat. No. 3 839 297.

Sutures and osteosynthesis devices which are manufactured of poly-β-hydroxybutyric acid (PHB)

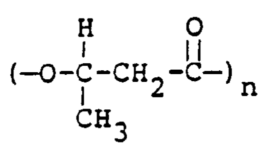

are described in G.B. Pat. No. 1 034 123.

Sutures and osteosynthesis devices which are manufactured of polydioxanone (PDS)

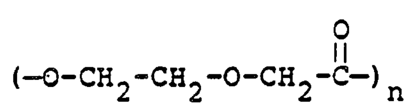

are described in U.S. Pat. No. 4 052 988.

Absorbable surgical devices which are manufactured about polyesteramides (PEA)

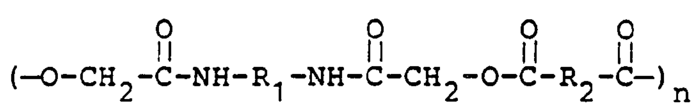

are described in U.S. Pat. No. 4 343 931.

Absorbable surgical devices of the above inventions are typically plates which are fixed to bone by screws,

cylindrical medullary nails or corresponding structures which are manufactured by melting absorbable polymer and by molding or pressing the melt into a suitable form. The mechanical strengths of such samples, which are manufactured by melt processing techniques, have typically the same order of magnitude as those of other similar synthetic polymers. Accordingly the tensile strengths of dry, unhydrolyzed samples manufactured about PGA, PLA, PHB and PGA/PLA have typically the order of magnitude of 40-80 MPa (see e.g. Kulkarni, R.K., Moore, E.G., Hegyeli, A.F. and Fred, L., J. Biomed. Mater. Res., 1971, 5, 169, Vert, M., Chabot, F. and Leray, J., Makromol. Chem. Suppl., 1981, 5, 30, Christel, P., Chabot, F., Leray, J.L., Morin, C. and Vert, M., in Biomaterials (Eds. G.D. Winter, D.F. Gibbons and H. Plenk, Jr.), Wiley (1980), p. 271, Tunc, D.C., Transactions of 9th Annual Meeting of the Society for Biomaterials, Birmingham, USA, 1983, p. 47, Howells, E.R., Chem. Ind., 1982, 7, 509).

The tensile strengths given above are modest if compared with tensile strengths of compact bone (ca. 80-200 MPa). Therefore the modest mechanical properties of the above materials have limited greatly their applications in surgical practice.

The initial mechanical strength of surgical absorbable osteosynthesis materials has been tried to improve by applying in absorbable polymeric matrix as reinforcement elements absorbable fibers with higher melting point than that of matrix itself e.g. polylactide matrix reinforced with polyglycolide fibers (in U.S. Pat. No. 4 279 249). Also biostable carbon fibers have been used as reinforcement elements. When the chemical element composition of reinforcement elements differs from that of matrix material the materials cannot form, as a rule, good adhesion between each others. Additionally, one consequence of the chemical differences of matrix polymer and reinforcement fibers is their different absorption rates in tissue conditions. On the other hand, when carbon fiber reinforcement is applied, carbon fiber remnant is left in tissues after the absorption of the matrix polymer.

Finnish patent application FI 851828 (=EP-A-0204 931) describes an osteosynthesis device or its component, which is self-reinforced, it means, it consists at least of the matrix of absorbable polymer or copolymer, which is reinforced with absorbable fibers or corresponding reinforcement elements which have the corresponding chemical element composition to that of the matrix.

The absorbable polymeric materials of the above invention are effectively self-reinforced, which gives materials with high initial mechanical strength and with pronounced chemical structure, which materials can be applied as surgical absorbable osteosynthesis devices or as their components like e.g. as osteosynthesis plates with screw fixation, as fixation screws or as stiff plates or bars.

Self-reinforecement means that the matrix polymer has been reinforced with fibers, film fibers, rods or corresponding reinforcement elements which have high tensile and/or shear strength or reinforcement is done with structures like threads, braids, bands, woven fabrics, non-woven fabrics etc. which are constructed of the above mentioned elements, in such a way that the matrix polymer and the reinforcement elements have the same chemical element percentual composition. In such a case it is possible to apply effectively the high initial strength of reinforcement elements like fibers (typically 500-2000 MPa) in manufacturing of macroscopical samples. When the fibers are bound together with a melt of polymer, copolymer or polymer mixture (matrix polymer) which has the same chemical composition as the fibers, the composite structure is achieved which has an excellent adhesion between the matrix and the reinforcement fibers and which therefore has also good mechanical properties.

Because the reinforcement elements of self-reinforced material contribute significantly to the strength of the material, decreases the strength of the whole material immediately after the decrease of the strength of the reinforcement elements. Self-reinforced materials are manufactured by transforming the material component, which acts as a matrix, by means of heat and/or pressure to such a physical state, which makes possible the development of adhesion between reinforcement elements and matrix. In practice this can be done in molds or dies, where the material is handled. When the matrix material is in a state of relatively small viscosity (in a following state) during pressing of the material, the matrix material flows from the surfaces of reinforcement elements which are in contact with the walls of the mold and therefore the reinforcement elements remain in a contact with the wall of the mold. On the other hand, when the matrix material is during molding process in a state of very high viscosity the effective wetting of all surfaces of reinforcement elements may encounter considerable difficulties. As a consequence of the above mentioned difficulties part of the surfaces of the reinforcement elements is typically bare on the surface of the sample after it has been removed from the mold. This fenomenon is well-known also more generally in manufacturing of fiber reinforced composites. Accordingly it is not possible in the case of the conventional self-reinforced materials to eliminate totally the possibility that part of fibers have a direct contact with the hydrolyzing environment. Additionally such partially bare fibers form on the surface of the material unfavorable stress concentrations which decreases the mechanical strength of the material. Both of the above mentioned factors decreases the initial mechanical strength and the hydrolysis resistance of the material. The direct contact of the reinforcement fibers to their environment has been eliminated in an invention FI Pat. Appl. 852706 (=W0 87/00059), which describes layered osteosynthesis devices or their parts, which have a flexible surface layer which protects a more stiff core. In an advantageous case the flexible surface layer can act also as a hydrolysis barrier, which protects the reinforcement elements of the self-reinforced structure. However, it is not possible, as a rule, to establish a good adhesion between the flexible surface polymer and stiff self-reinforced core because of their different mechanical states. Such a poor adhesion is seen e.g. as loosening of the surface layer from the core in hydrolytic conditions.

In this invention we have found unexpectedly that when the self-reinforced material is coated with a coating layer, which has the same chemical element percentual composition as the matrix polymer and reinforcement elements have, we accomplish self-reinforced coated (layered) materials, which have better initial strength properties and better retention of mechanical strength in hydrolytic conditions in vivo and in vitro than the known non-coated self-reinforced materials and/or the known coated materials with self-reinforced core. This observation is unexpected and surprising especially therefore that even the surface layer which is manufactured of comparatively rapidly absorbable material (like of polyglycolic acid) which is fixed on the surface of self-reinforced core with the same chemical composition, causes for the material better strength and/or strength retention in hydrolytic conditions than the surface layer that degrades more slowly than the core but which has a chemical composition that differs from that of the core (e.g. polydioxanone layer on the surface of self-reinforced polyglycolic acid). The materials of this invention can be applied to manufacture surgical fixation devices which have better strength and strength retention in hydrolytic conditions and which therefore are also more secure for patients than the earlier known self-reinforced devices and materials.

When the self-reinforced material structure is covered with a surface layer, which has the same chemical element percentual composition as the self-reinforced core, an excellent adhesion is created between the surface layer and the core. In such a case the positive effect of the coating upon the strength properties of the material is at its best. A compact, homogeneous surface layer contributes to the mechanical properties of the material in several days. At first the coating covers the bare parts of reinforcement elements and eliminates in this way from the surface of the self-reinforced structure the stress concentrations which could decrease the mechanical strength of the material. Secondly the coating slackens the diffusion of water and dissolved material into the self-reinforced structure by improving in this way the hydrolysis resistance of the material. Further, the materials of this invention have excellent adhesion between coating and self-reinforced core. Therefore the coating does not loosen in hydrolytic conditions like such coatings as a rule loosen which have a poor adhesion to the core as a consequence of different chemical element percentual composition.

The materials of this invention can be applied to manufacture different absorbable fixation devices or their parts or components to different surgical purposes for fixation of different tissues or parts of tissues to each others by techniques of internal fixation or external fixation. Such typical devices of this invention are e.g. rods, plates, screws, nails, intramedullary nails, clamps, cramps etc., which can be applied in internal and/or external fixation of bone fractures, osteotomies, arthrodesis, joint damages and/or of cartilage tissue. Further we can mention staples, clamps, plates, cramps and corresponding devices, which can be applied in fixation of damaged and/or operated soft tissues, fasciae, organs etc. to each other. Further we can mention cords, ribbons and corresponding, which can be applied to support damaged or operated tendons and ligaments. Further such devices are plates, chutes and matrices, which can be applied in reconstruction and augmentation of bone and cartilage tissue, further also different tube-like or ring-like devices, which can be applied in joining blood vessels, nerves etc. oblong tissues to each other. Further the materials of this invention can be applied as combined with different ceramic materials to biocomposites e.g. by replacing the self-reinforced components of biocomposites of the invention FI Pat. Appl. 864457 with materials of this invention. It is also possible to manufacture self-reinforced layered materials and devices to different applications from different absorbable polymers, copolymers and polymer mixtures. In Table 1 are given some absorbable polymers which can be applied in manufacturing of the materials and devices of this invention.

It is natural that also other absorbable polymers than those been given in Table 1 can be applied in manufacturing of materials of this invention.

Figure 1 shows schematically the structure of a typical material of this invention.

Self-reinforced, coated materials of this invention can be manufactured e.g. by making first the self-reinforced core e.g. by some method given in the invention FI Pat. Appl. 851828 and by coating the self-reinforced core with a melt of the same polymer or with a solution of the same polymer and by evaporating the solvent.

It is self-evident that the self-reinforced coated material can contain in addition to the above mentioned components also different additives, like colours, ceramic powders, antibiotics, enzymes, proteins etc. additives, which can give to the material special functional advantages or which can change the processing or the properties of the material.

The following non-limiting examples illustrate the present invention.

EXAMPLE 1.

Polyglycolic acid sutures (Dexon®, size 2 USP) were

Table 1. Absorbable polymers

| Polymer |
|---|
| Polyglycolide (PGA) |
| Copolymers of glycolide:<br>Glycolide/L-lactide copolymers (PGA/PLLA)<br>Glycolide/trimethylene carbonate copolymers (PGA/TMC) |
| Polylactides (PLA) |
| Stereocopolymers of PLA:<br>Poly-L-lactide (PLLA)<br>Poly-DL-lactide (PDLLA)<br>L-lactide/DL-lactide copolymers |
| Copolymers of PLA:<br>Lactide/tetramethylglycolide copolymers<br>Lactide/trimethylene carbonate copolymers<br>Lactide/δ-valerolactone copolymers<br>Lactide/ε-caprolactone copolymers<br>Polydepsipeptides<br>PLA/polyethylene oxide copolymers<br>Unsymmetrically 3,6-substituted poly-1,4-dioxane-2,5-diones |
| Poly-β-hydroxybutyrate (PHBA)<br>PHBA/β-hydroxyvalerate copolymers (PHBA/HVA) |
| Poly-β-hydroxypropionate (PHPA) |
| Poly-p-dioxanone (PDS) |
| Poly-δ-valerolactone |
| Poly-ε-caprolactone |
| Methylmethacrylate-N-vinyl pyrrolidone copolymers |
| Polyesteramides |
| Polyesters of oxalic acid |
| Polydihydropyrans |
| Polyalkyl-2-cyanoacrylates |
| Polyurethanes (PU) |
| Polyvinylalcohol (PVA) |
| Polypeptides |
| Poly-β-malic acid (PMLA) |
| Poly-β-alkanoic acids |

Reference: P. Törmälä, S. Vainionpää and P. Rokkanen in IVA's Beijer Symposium "Biomaterials and Biocompatibility", Stockholm, Sweden, August 25-26, 1987.

heated in a pressurized cylindrical mold (the length 50 mm, diameter ∅ 3.2 mm) at the temperature of 218°C in a nitrogen atmosphere 5 min with a pressure of 2000 bar. The softened fiber material was sintered partially together. The mold was cooled to room temperature rapidly. The bending strength of self-reinforced absorbable composite rods which were manufactured with the above method (specimen series 1) was 340 MPa. Corresponding self-reinforced, coated rods were manufactured by coating self-reinforced cores in a heated, evacuated, cylindrical mold (the length 50 mm, ∅ 3.4 mm) with a melt of polyglycolic acid ($M_w$ = 140.000) and by cooling rapidly the mold. These self-reinforced, coated, absorbable composite rods (specimen series 2) had a bending strength of 380 MPa.

To a reference material were selected self-reinforced rods of specimen series 1 which were coated with polydioxanone (PDS) melt ($M_w$ = 20.000) in a heated, evavuated, cylindrical mold (the length 50 mm, ∅ 3.4 mm) and by cooling rapidly the mold. The PDS coated rods (specimen series 3) had the bending strength of 360 MPa.

The hydrolysis behaviour of the above mentioned rods was studied in vitro and in vivo.

In vitro

The rods were hydrolyzed in distilled water at 37°C 3 weeks and their bending strengths were measured. The following bending strengths values were obtained (mean values of 5 measurements):

Specimen series 1: 200 MPa
Specimen series 2: 300 MPa
Specimen series 3: 230 MPa

The PDS coating of some rods of specimen series 3 had loosened at some spots which was seen as bubble-like formations on the surface of the rods.

In vivo

Rods were implanted subcutaneously into the backs of rabbits for 3 weeks. The methods and techniques of implantations and removal operations of rods were those given in the publication S. Vainionpää: Thesis, Helsinki University, Helsinki, Finland, 1987. After removal the implanted rods were stored immediately in a physiological saline solution and their bending strengths were measured during the same day. The following values were obtained for bending strengths (mean values of 5 measurements):

Specimen series 1: 110 MPa
Specimen series 2: 150 MPa
Specimen series 3: 110 MPa.

The PDS -coating of rods of specimen series 3 was clearly loosened from the self-reinforced core.

The above mentioned in vitro and in vivo hydrolysis studies showed that the materials of this invention (specimen series 2) have clearly better strength and hydrolysis resistance than the corresponding earlier known materials.

EXAMPLE 2.

Self-reinforced rods of specimen series 1 of Example 1 were coated in a heated, evacuated, cylindrical mold (the length 50 mm, ∅ 3.4 mm) with a melt of polyglycolic acid ($M_w$ = 250.000) and by cooling rapidly the mold. The rods were hydrolyzed in distilled water at 37°C 3 weeks and their bending strengths were measured. These self-reinforced, coated PGA rods with a high molecular weight coating showed 6 % higher bending strength than the rods of specimen series 2 of Example 1.

EXAMPLE 3.

Self-reinforced rods of specimen series 1 of Example 1 were heated in a pressurized, evacuated, cylindrical mold (the lengt 50 mm, ∅ 3.2 mm) at a temperature about 225°C for 30 seconds and the mold was cooled rapidly to room temperature. This treatment formed a thin skin-like, nonfibrous coating on the surface of the rods. The rods were hydrolyzed in distilled water at 37°C 3 weeks and their bending strengths were measured. These self-reinforced, coated PGA rods showed the bending strength of 230 MPa (the mean value of 5 measurements).

EXAMPLE 4.

Fibers were manufactured by a melt spinning process of a polymer mixture comprising 50 % poly-L-lactide (PLLA, $M_w$ = 250.000) and 50 % poly-D-lactide (PDLA, $M_w$ = 250.000) (a polymer mixture described in a Japanese Pat. Appl. 61-36321). The manufactured fibers had following properties: melting point 232°C, fiber diameter 40 μm and tensile strength 440 MPa.

The above fibers were applied in manufacturing of absorbable reinforced, coated composite rods in the following way. First the fibers were melt-coated with a thin surface layer of absorbable polymer by drawing the fibers through the melt of an absorbable polymer in such a way that the weight ratio of fibers to the coating was 60/40. The following coatings were used: (1) PLLA ($M_w$ 250.000), (2) PDLLA ($M_w$ 100.000), (3) a mixture of PLLA ($M_w$ 250.000) and PLLA ($M_w$ 100.000) in a ratio of 1:1, (4) PGA ($M_w$ 40.000). The coated fiber samples were compression molded to rods (samples 1-4) with the length 50 mm and ∅ 3.2 mm at temperatures 180°C - 278°C in the first mold of Example 1. Additionally the rods were coated with a 0.1 mm thick layer of the melt of the coating polymer by the method given in Example 1. The bending strengths of the rods were following (the values for non-coated cores are given in parentheses): (1) 300 (260), (2) 240 (220), (3) 285 (250) and (4) 240 (225). The numbers (1) - (4) refer to the coated raw-material fibers.

EXAMPLE 5.

The self-reinforced, coated rods of specimen series 2 of Example 1 were cut from both ends by a dentists diamond saw by sawing a 2 mm long piece away from both ends of the rod. This exposed the core material of rods from its both ends. The cut rods, and non-cut rods (of specimen series 2) as a reference, were hydrolyzed in distilled water at 37°C 3 weeks and the bending strengths of rods were measured. The cut rods showed equal bending strengths (ca. 300 MPa) with the non-cut rods. The shear strengths of coated, cut and non-cut rods (specimen series 2 of Example 1) were also measured after 3 weeks hydrolysis from different parts of the rods. The non-cut rods showed a mean shear strength of 120±10 MPa when measured at 3, 10, 20, 30 and 40 mm distance from the other end of the rod. The cut rods showed the same shear strength when it was measured at a distance which was 4 mm or more from the cut ends of the rod. The shear strength of the ends of the cut rods at a distance of 4 mm or less from the end was after hydrolysis lower (< 160±20 MPa) than the shear strength in the middle region of rods (ca. 210 MPa). This showed that the removal of coating from the ends of the rods increased somewhat the diffusion rate of water into the structure of rod. However, the cut, partially coated rods were of their shear strength and bending strength superior to corresponding non-coated rods (rods of specimen series 1 of Example 1) which after 3 weeks hydrolysis showed bending strength 200 MPa and shear strength 130±20 MPa.

The studies of this example showed that even partial coating of the surface of self-reinforced material improves the strength retention of the material in hydrolytic conditions.

## Claims

1. Self-reinforced, absorbable materials and devices or their parts or components for surgical fixation of damaged and/or operated, tissues, which materials, devices or their parts or components comprise:
   (1) matrix of an absorbable polymer or copolymer or of a polymer mixture,
   (2) reinforcement elements or structures constructed of them which are at least partially embedded in matrix and which reinforcement elements have the same chemical element percentual composition as matrix has and
   (3) a coating layer which covers a substantial part of the surface of the matrix-reinforcement element system and which coating layer has the same chemical element percentual composition as matrix (1) and reinforcement elements (2) have.

## Patentansprüche

1. Selbstverstärkte, absorbierbare Materialien und Vorrichtungen oder deren Teile oder Bestandteile zur chirurgischen Befestigung von beschädigten und/oder operierten Geweben, wobei die Materialien, Vorrichtungen oder deren Teile oder Bestandteile enthalten:
   (1) eine Matrix aus einem absorbierbaren Polymer oder Copolymer oder Polymergemisch,
   (2) Verstärkungselemente oder daraus aufgebaute Strukturen, welche wenigstens teilweise in die Matrix eingebettet sind und wobei die Verstärkungselemente die gleiche prozentuale chemische Elementzusammensetzung wie die Matrix aufweisen,
und
(3) eine Beschichtung, welche einen wesentlichen Teil der Oberfläche des matrixverstärkten Elementsystems bedeckt und die gleiche prozentuale chemische Elementzusammensetzung aufweist, wie die Matrix (1) und die Verstärkungselemente (2).

## Revendications

**1.** Matières et dispositifs absorbables autorenforcés ou leurs parties ou constituants pour la fixation chirurgicale de tissus endommagés et/ou opérés, caractérisés en ce que ces matières, dispositifs ou leurs parties ou composants comprennent :
(1) une matrice d'un polymère ou copolymère ou d'un mélange de polymères absorbable,
(2) des éléments ou structures de renforcement construits en ceux-ci qui sont au moins partiellement enrobés dans la matrice et qui ont la même composition centésimale élémentaire chimique que la matrice et
(3) une couche de revêtement qui recouvre une partie substantielle de la surface du système d'éléments de renforcement de la matrice et qui a la même composition centésimale élémentaire chimique que la matrice (1) et que les éléments de renforcement (2).

SELF-REINFORCED COATED STRUCTURE

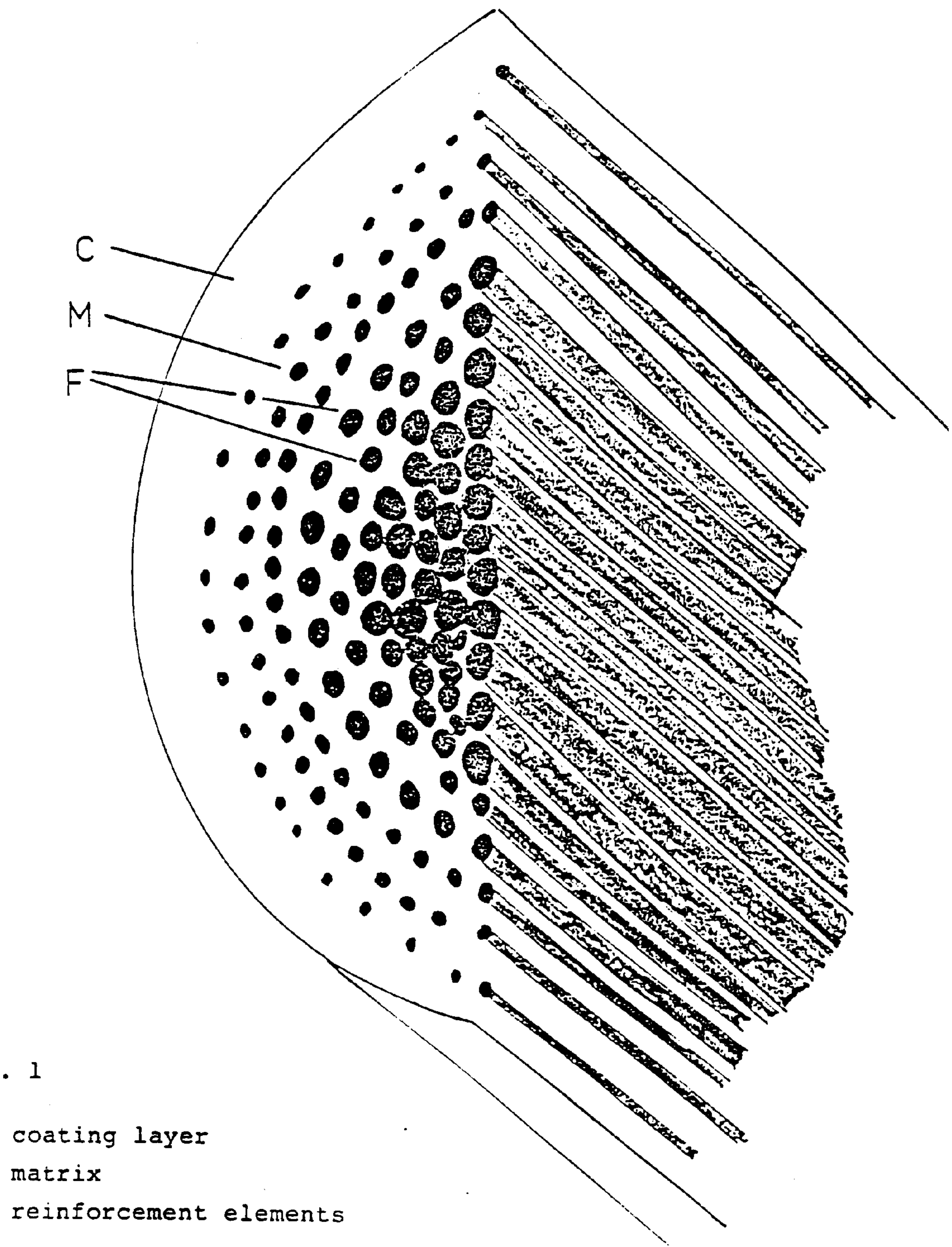


FIG. 1

C = coating layer

M = matrix

F = reinforcement elements